# EUROPEAN PATENT APPLICATION

(11) **EP 4 353 175 A1**
(43) Date of publication of application: **17.04.2024**
(21) Application number: 22212817.5
(22) Date of filing: 12.12.2022
(51) Int. Cl.: A61B 34/10

(54) **PROVIDING GUIDANCE FOR A TREATMENT PROCEDURE ON AN OCCLUDED VESSEL**

(30) Priority: 11.10.2022 US 202263415034 P
(71) Applicant: Koninklijke Philips N.V., 5656 AG Eindhoven (NL)
(72) Inventor: NICKISCH, Hannes, Eindhoven (NL); GRASS, Michael, Eindhoven (NL); HAASE, Hans Christian, 5656AG Eindhoven (NL); VAN DER HORST, Arjen, Eindhoven (NL); SCHMITT, Holger, Eindhoven (NL); VEMBAR, Manindranath, Eindhoven (NL)
(74) Representative: Philips Intellectual Property & Standards

(57) **Abstract**

A computer-implemented method of providing guidance for a treatment procedure on an occluded vessel, is provided. The method includes: analyzing CT data to determine one or more properties of an occlusion in the vessel; determining, based on the one or more properties, a recommended intravascular treatment device for treating the occluded vessel; and outputting an indication of the recommended intravascular treatment device.

## Description

### TECHNICAL FIELD

The present disclosure relates to providing guidance for a treatment procedure on an occluded vessel. A computer-implemented method, a computer program product, and a system, are disclosed.

### BACKGROUND

Blood vessels in the anatomy may become blocked, or occluded, for various reasons. For instance, occluded blood vessels may result from the build-up of plaque in the vessel, or from a clot in the vessel, also known as a thrombosis. Occlusions can occur in blood vessels in various parts of the anatomy, including in the heart, in the brain, and in peripheral regions such as the leg.

In some cases an occlusion is partial, and blood flow in a vessel is restricted by an occlusion but not inhibited. For instance, in the heart, a partial occlusion of a coronary artery may result in the heart having to work harder in order to maintain blood flow. A subject with a partial occlusion of a coronary artery might suffer from chest pain, or angina. However, in more severe cases, blood flow in a vessel may be completely inhibited by an occlusion. A chronic total occlusion, or "CTO" is defined as a complete blockage of a blood vessel for a duration of greater than or equal to 3 months. The lack of blood flow arising from a coronary CTO can result in permanent damage to the heart muscle. CTOs are typically caused by a combination of severe atherosclerosis, and thrombosis. Atherosclerosis is a condition which plaques that are made up of fat, cholesterol, calcium, fibrin, and other substances build-up in the walls of arteries. These plaques cause the arteries to harden and narrow, restricting the blood flow and supply of oxygen. Over time, a plaque may open, or rupture, forming a thrombus, or blood clot, that further restricts the blood flow. The thrombosis, in combination with the plaque, can completely block the artery, resulting in a CTO.

Occlusions are often treated using a procedure known as atherectomy; a minimally-invasive endovascular procedure that is used to remove atherosclerosis from blood vessels within the body, and thereby restore blood flow. Various types of intravascular treatment devices are available for treating occlusions during atherectomy procedures. A review of such devices is described in a document by Dash, D., et al., "Contemporary treatment options for surmounting the conundrum of calcified coronaries", Journal of Transcatheter Interventions., 2020;28:eA202007. The types of intravascular treatment devices that are currently available include: a laser atherectomy device that emits laser irradiation to break-up plaque, a directional atherectomy device that includes an inflatable balloon and a cutting window for removing occluding material from the vessel wall, a rotational atherectomy device that includes a burr that is rotated in order to perform a rotational sanding or cutting operation that breaks-up plaque, an orbital atherectomy device that includes an eccentrically-mounted abrasive crown to perform an orbital sanding operation to break-up plaque, a transluminal atherectomy device that includes a rotating blade and an aspirator that respectively excise and aspirate atheroma, and an IVL balloon that delivers shock wave pulses to the vessel in order to break-up plaque.

An example of a commercial laser atherectomy device is the Turbo-Elite laser atherectomy catheter marketed by Philips Healthcare, Best, The Netherlands. An example of a commercial directional atherectomy device is the HawkOne Directional Atherectomy System marketed by Medtronic, Minneapolis, USA. An example of a commercial rotational atherectomy device is the Rotapro rotational atherectomy system marketed by Boston Scientific, Massachusetts, USA. An example of a commercial orbital atherectomy device is the Diamondback 360 Coronary Orbital Atherectomy System marketed by Cardiovascular Systems Inc., Minneapolis, USA. An example of a commercial transluminal atherectomy device is the AngioJet Thrombectomy System marketed by Boston Scientific, Massachusetts, USA. An example of a commercial IVL balloon is the Shockwave C2 Coronary IVL Catheter marketed by Shockwave Medical, Santa Clara, USA.

However, there remains a need to provide improved guidance for occlusion treatment procedures. The properties of occlusions vary in terms of their size, shape, and composition, and consequently the intravascular treatment devices that are available for treating occlusions have different strengths in relation to these properties. It can therefore be challenging to select a type of treatment device for use in an occlusion treatment procedure. The type of the device that is used to treat an occlusion can influence factors such as the ease of performing the procedure, the effectiveness of the treatment, and consequently its outcome. Furthermore, having selected a treatment device to treat an occlusion, it can be challenging to determine the properties of the device to use in the procedure. Properties of the device such as its stiffness, and its tip shape, can influence the effectiveness of the treatment, and consequently its outcome. It can also be challenging to determine the values of treatment parameters to use with the device. For instance, if the device is a laser atherectomy catheter, the values of parameters such as a fluence of optical irradiation emitted by the device, and the rate of its advancement in the artery, may need to be adjusted in order to optimize the desired outcome.

### SUMMARY

According to one aspect of the present disclosure, a computer-implemented method of providing guidance for a treatment procedure on an occluded vessel, is provided. The method includes:
receiving computed tomography, CT, data representing the occluded vessel;
analyzing the CT data to determine one or more properties of an occlusion in the vessel;
determining, based on the one or more properties, a recommended intravascular treatment device for treating the occluded vessel; and
outputting an indication of the recommended intravascular treatment device.

In the above method, the recommended intravascular treatment device is determined based on a property/ properties of the occlusion that are determined from CT data for the occluded vessel. Consequently, it is ensured that the recommended intravascular treatment device is suited to the occlusion in the vessel. Thus, a more effective treatment of the occluded vessel may be realized.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating examples of various properties of a CTO in accordance with some aspects of the present disclosure: A) Size: short length, B) Size: long length, C) Shape: >45 degrees of bend at CTO entry, D) Shape: >45 degrees of bend along CTO path, E) Entry shape: tapered cap, F) Entry shape: blunt cap, G) Composition: Calcification along CTO.
Fig. 2 is a schematic diagram illustrating examples of various intravascular treatment devices for treating occlusions, in accordance with some aspects of the present disclosure: A) Laser atherectomy, B) Directional atherectomy, C) Rotational atherectomy, D) Orbital atherectomy E) Transluminal atherectomy, E) IVL balloon.
Fig. 3 is a flowchart illustrating an example of a computer-implemented method of providing guidance for a treatment procedure on an occluded vessel, in accordance with some aspects of the present disclosure.
Fig. 4 is a schematic diagram illustrating a first example of a system 200 for providing guidance for a treatment procedure on an occluded vessel, in accordance with some aspects of the present disclosure.
Fig. 5 is a schematic diagram illustrating a second example of a system 200 for providing guidance for a treatment procedure on an occluded vessel, in accordance with some aspects of the present disclosure.

### DETAILED DESCRIPTION

Examples of the present disclosure are provided with reference to the following description and Figs. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to examples of a computer-implemented method of providing guidance for a treatment procedure on an occluded vessel. In some of the examples, the occlusion in the vessel is a CTO. Thus, the method disclosed herein may be used with occluded vessels in which the occlusion is a CTO. However, it is to be appreciated that the method disclosed herein may be used with occluded vessels in which there are other types of occlusions. In other words, the method may be used with occluded vessels in general.

Reference is also made herein to examples in which the method is used to provide guidance for a treatment procedure on an occluded vessel in which the vessel is a coronary artery. However, it is also to be appreciated that the vessel may in general be any type of vessel in the anatomy. Thus, the vessel may be an artery, or a vein, and the artery, or vein, may be in any location in the body, such as in the heart, the brain, the arm, the leg, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

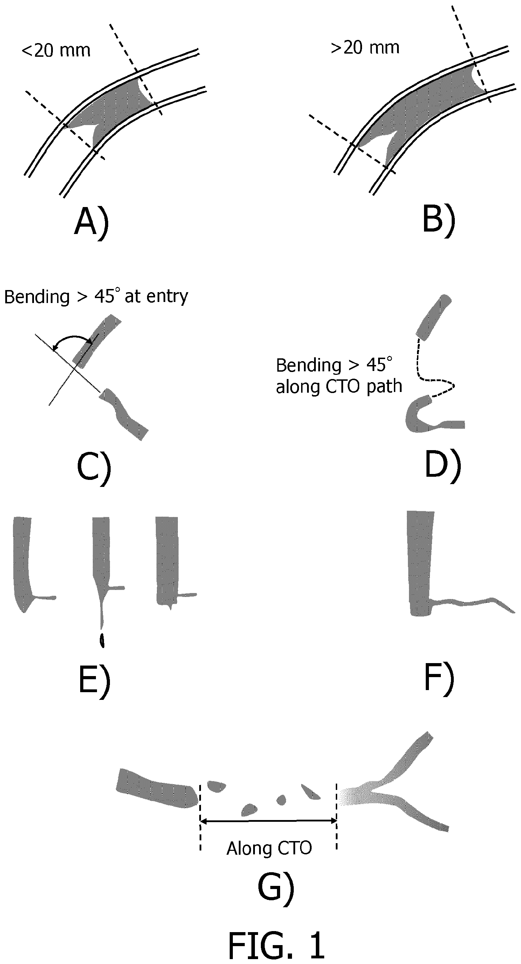

As mentioned above, there remains a need to provide improved guidance for occlusion treatment procedures. The properties of occlusions vary in terms of their size, shape, and composition, and consequently the intravascular treatment devices that are available for treating occlusions have different strengths in relation to these properties. By way of an example, Fig. 1 is a schematic diagram illustrating examples of various properties of a CTO in accordance with some aspects of the present disclosure: A) Size: short length, B) Size: long length, C) Shape: >45 degrees of bend at CTO entry, D) Shape: >45 degrees of bend along CTO path, E) Entry shape: tapered cap, F) Entry shape: blunt cap, G) Composition: Calcification along CTO.

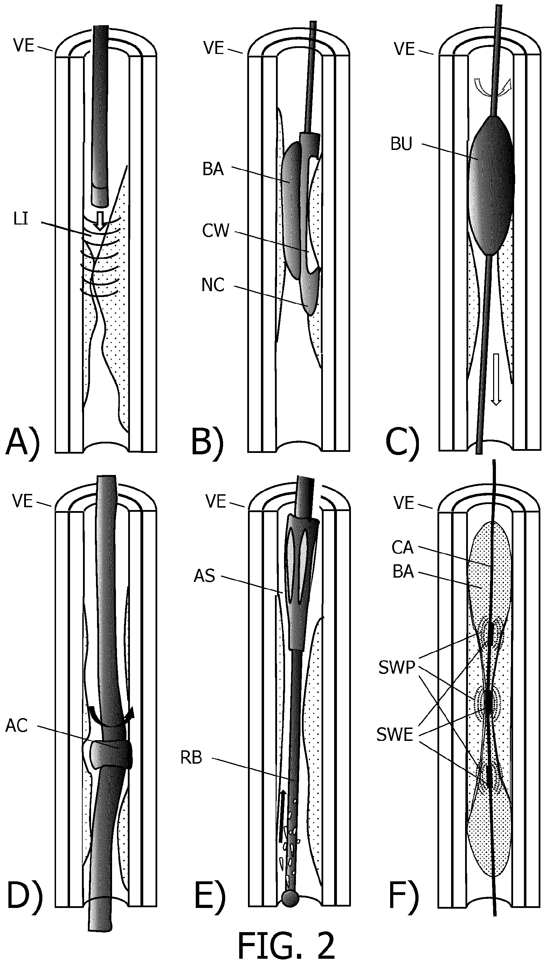

Fig. 2 is a schematic diagram illustrating examples of various intravascular treatment devices for treating occlusions, in accordance with some aspects of the present disclosure: A) Laser atherectomy, B) Directional atherectomy, C) Rotational atherectomy, D) Orbital atherectomy E) Transluminal atherectomy, E) IVL balloon. The laser atherectomy device illustrated in Fig. 2A emits laser irradiation LI to break-up plaque via photoablation. The directional atherectomy device illustrated in Fig. 2B includes an inflatable balloon BA and a cutting window CW. The cutting window CW is rotated within the vessel and aligned with occluding material on the vessel wall. In this position the inflatable balloon is expanded in order to stabilize the cutting window CW and to press the cutting window against the occluding material. The cutting blade is then rotated and translated, which both removes the occluding material from the vessel wall, as well as packs the removed material into the nosecone NC of the device so that it can be removed from the vessel. The rotational atherectomy device illustrated in Fig. 2C includes a burr with a roughened surface. The burr is rotated in order to perform a rotational sanding or cutting operation that breaks-up plaque. The orbital atherectomy device illustrated in Fig. 2D includes an eccentrically-mounted abrasive crown that uses centrifugal force to perform an orbital sanding operation that breaks-up plaque. The transluminal atherectomy device illustrated in Fig. 2E includes a rotating blade RB and an aspirator AS that respectively excise, and aspirate, atheroma. The IVL balloon illustrated in Fig. 2F includes one or more shock wave emitters SWE that are arranged on a catheter CA. The IVL balloon operates by delivering shock wave pulses SWP from the shock wave emitter(s) SWE to the vessel VE in order to break-up plaque. In-use, the balloon BA is filled with a liquid such as saline in order to expand the balloon and provide acoustic impedance matching between the emitter(s) EM and the vessel.

As may be appreciated from the from the range of properties of occlusions that are illustrated in Fig. 1 and the different means of action of the types of treatment devices for treating occlusions that are illustrated in Fig. 2, the intravascular treatment devices that are available for treating occlusions have different strengths in relation to the properties of occlusions. Consequently, the type of treatment device that is used to treat an occlusion can influence factors such as the ease of performing the procedure, the effectiveness of the treatment, and its outcome. Thus, it can be challenging to select a type of treatment device for use in an occlusion treatment procedure.

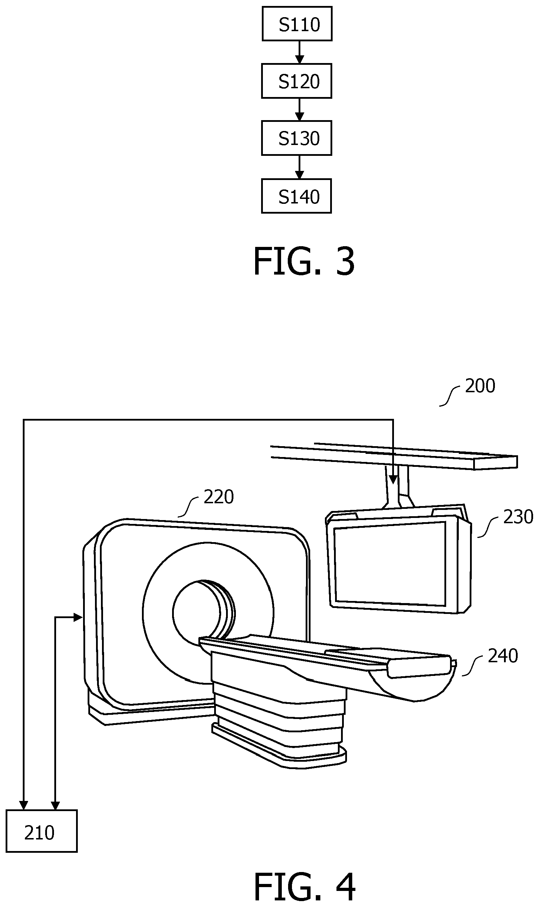

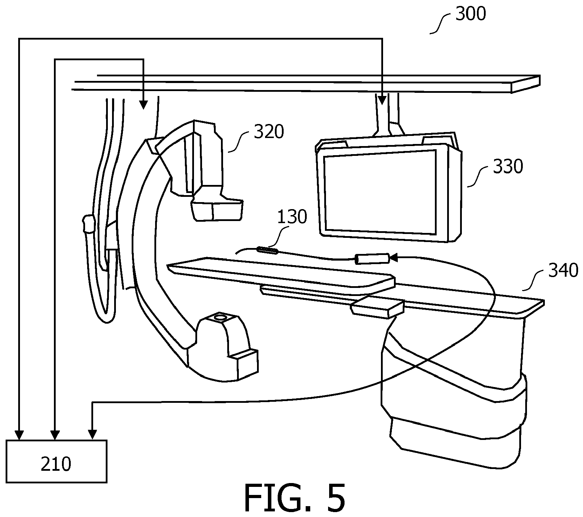

Fig. 3 is a flowchart illustrating an example of a computer-implemented method of providing guidance for a treatment procedure on an occluded vessel, in accordance with some aspects of the present disclosure. Fig. 4 is a schematic diagram illustrating a first example of a system 200 for providing guidance for a treatment procedure on an occluded vessel, in accordance with some aspects of the present disclosure. Fig. 5 is a schematic diagram illustrating a second example of a system 200 for providing guidance for a treatment procedure on an occluded vessel, in accordance with some aspects of the present disclosure. The systems 200, 300, each include one or more processors 210. It is noted that operations described in relation to the method illustrated in Fig. 3, may also be performed by the one or more processors 210 of the systems 200, 300 illustrated in Fig. 4 and in Fig. 5. Likewise, operations described in relation to the one or more processors 210 of the systems 200, 300 may also be performed in the method described with reference to Fig. 3.

With reference to Fig. 3, the computer-implemented method of providing guidance for a treatment procedure on an occluded vessel, includes:
receiving S110 computed tomography, CT, data representing the occluded vessel 110;
analyzing S120 the CT data to determine one or more properties of an occlusion 120 in the vessel;
determining S130, based on the one or more properties, a recommended intravascular treatment device 130_{1..6} for treating the occluded vessel 110; and
outputting S140 an indication of the recommended intravascular treatment device 130_{1..6}.

In the above method, the recommended intravascular treatment device is determined based on a property/ properties of the occlusion that are determined from CT data for the occluded vessel. Consequently, it is ensured that the recommended intravascular treatment device is suited to the occlusion in the vessel. Thus, a more effective treatment of the occluded vessel may be realized.

With reference to the method illustrated in Fig. 3, in the operation S110, CT data 140 representing the occluded vessel, is received.

In general, the CT data that is received in the operation S110 may represent a static image of the occluded vessel, or alternatively it may represent a temporal sequence of images of the occluded vessel. In the latter case, the temporal sequence of images may be generated substantially in real-time, and the method described with reference to Fig. 3 may be performed substantially in real-time. Consequently, in one example, the indication of the recommended intravascular treatment device may be outputted in real-time.

In general, the CT data that is received in the operation S110 may be raw data, i.e. data that has not yet been reconstructed into a volumetric, or 3D, image, or it may be image data, i.e. data that has already been reconstructed into a volumetric image. The CT data may also be referred-to as volumetric data. The CT data may be generated by a CT imaging system, or, as described in more below, alternatively it may be generated by rotating, or stepping the X-ray source and X-ray detector of an X-ray projection imaging system around the vessel.

A CT imaging system generates CT data by rotating, or stepping, an X-ray source-detector arrangement around an object and acquiring X-ray attenuation data for the object from multiple rotational angles with respect to the object. The CT data may then be reconstructed into a 3D image of the object. Examples of CT imaging systems that may be used to generate the CT data include cone beam CT imaging systems, photon counting CT imaging systems, dark-field CT imaging systems, and phase contrast CT imaging systems. An example of a CT imaging system 220 that may be used to generate the CT data that is received in the operation S110, is illustrated in Fig. 4. By way of an example, the CT data may be generated by the CT 5000 Ingenuity CT imaging system that is marketed by Philips Healthcare, Best, The Netherlands.

As mentioned above, the CT data that is received in the operation S 110 may alternatively be generated by rotating, or stepping the X-ray source and X-ray detector of an X-ray projection imaging system around the vessel. An X-ray projection imaging system typically includes a support arm such as a so-called "C-arm" that supports an X-ray source and an X-ray detector. X-ray projection imaging systems may alternatively include a support arm with a different shape to this example, such as an O-arm, for example. In contrast to a CT imaging system, an X-ray projection imaging system generates X-ray attenuation data for an object with the X-ray source and X-ray detector in a static position with respect to the object. The X-ray attenuation data may be referred-to as projection data, in contrast to the volumetric data that is generated by a CT imaging system. The X-ray attenuation data that is generated by an X-ray projection imaging system is typically used to generate a 2D image of the object. However, an X-ray projection imaging system may generate CT data, i.e. volumetric data, by rotating, or stepping, its X-ray source and X-ray detector around an object and acquiring projection data for the object from multiple rotational angles with respect to the object. Image reconstruction techniques may then be used to reconstruct the projection data that is obtained from the multiple rotational angles into a volumetric image in a similar manner to the reconstruction of a volumetric image using X-ray attenuation data that is acquired from a CT imaging system. Thus, the CT data that is received in the operation S 110, may be generated by a CT imaging system, or alternatively, it may be generated by an X-ray projection imaging system. An example of an X-ray projection imaging system that may be used to generate the CT data, is the Azurion 7 X-ray projection imaging system that is marketed by Philips Healthcare, Best, The Netherlands.

In some examples, the CT data that is received in the operation S110 is spectral CT data. Spectral CT data defines the X-ray attenuation of an object in a plurality of different energy intervals DE_{1..m}. In general there may be two or more energy intervals; i.e. *m* is an integer, and *m* ≥ 2. In this regard, the spectral CT data that is received in the operation S 110 may be generated by a spectral CT imaging system, or by a spectral X-ray projection imaging system. In the latter case, the spectral CT data may be acquired by rotating, or stepping the X-ray source and X-ray detector of the spectral X-ray projection imaging system around the vessel, as described above. More generally, the spectral CT data that is received in the operation S110 may be generated by a spectral X-ray imaging system.

The ability to generate X-ray attenuation data in multiple different energy intervals DE_{1..m} distinguishes a spectral X-ray imaging system from a conventional X-ray imaging system that generates X-ray attenuation data in a single energy interval. By processing the data from the multiple different energy intervals, a distinction can be made between media that have similar X-ray attenuation values when measured within a single energy interval, and which would be indistinguishable in X-ray attenuation data generated by a conventional X-ray imaging system. Examples of spectral X-ray imaging systems that may be used to generate spectral CT data that is received in the operation S 110 include cone beam spectral X-ray imaging systems, photon counting spectral X-ray imaging systems, dark-field spectral X-ray imaging systems, and phase contrast spectral X-ray imaging systems. An example of a spectral CT imaging system that may be used to generate spectral CT data that is received in the operation S110 is the Spectral CT 7500 that is marketed by Philips Healthcare, Best, The Netherlands.

In general, spectral CT data may be generated by various different configurations of spectral X-ray imaging systems that include an X-ray source and an X-ray detector. The X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval DE_{1..m}, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and detectors may be used to detect X-rays within different X-ray energy intervals DE_{1..m}. In general, discrimination between different X-ray energy intervals may be provided at the source by temporally switching the X-ray tube potential of a single X-ray source, i.e. "rapid kVp switching", or by temporally switching, or filtering, the emission of X-rays from multiple X-ray sources. The temporal switching may be performed within a rotation of the X-ray source-detector arrangement such that X-ray attenuation data for multiple different X-ray energy intervals DE_{1..m}, is acquired within a rotation; or alternatively, X-ray attenuation data for an energy interval may be acquired for an X-ray energy interval during a specified number of rotations of the gantry before switching to another energy interval and similarly acquiring X-ray attenuation data for that energy interval. In such configurations, a common X-ray detector may be used to detect X-rays across multiple different energy intervals, X-ray attenuation data for each energy interval being generated in a time-sequential manner. Alternatively, discrimination between different X-ray energy intervals may be provided at the detector by using a multi-layer detector, or a photon counting detector. Such detectors can detect X-rays from multiple X-ray energy intervals DE_{1..m} near-simultaneously, and thus there is no need to perform temporal switching at the source. Thus, a multi-layer detector, or a photon counting detector, may be used in combination with a polychromatic source to generate X-ray attenuation data at different X-ray energy intervals DE_{1..m}.

Other combinations of the aforementioned X-ray sources and detectors may also be used to provide the spectral CT data. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral CT data for different energy intervals DE_{1..m} is facilitated by virtue of the rotational offsets of the source-detector pairs. Improved separation between the spectral CT data for different energy intervals DE_{1..m}, may be achieved with this configuration by applying an energy-selective filter to the X-ray detector(s) in order to reduce the effects of X-ray scatter.

In general, the CT data that is received in the operation S110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The CT data that is received in the operation S110 may be received from various sources. For example, the CT data may be received from an imaging system, such as one of the imaging systems described above. Alternatively, the CT data may be received from another source, such as a computer readable storage medium, the Internet, or the Cloud, for example.

In the operation S120, the CT data is analyzed in order to determine one or more properties of an occlusion 120 in the vessel. In this regard, the one or more properties of the occlusion 120 may include properties such as a measurement of a size of the occlusion, a location of the occlusion, a measurement of a shape of the occlusion, a measurement of an entry shape of the occlusion, and a composition of the occlusion. Properties of the occlusion such as these can impact the choice of intravascular treatment device for treating the occlusion. Some examples of properties of the occlusion that may be determined in the operation S120 are illustrated in Fig. 1. In general, the measurement of a size of the occlusion may include a measurement of a length, or a diameter, or an area, or a volume, of the occlusion. Examples of measurements of the length of an occlusion are illustrated in Fig. 1A and Fig. IB. In general, the location of the occlusion may be defined in terms of the vessel in which the occlusion is located (e.g. left coronary artery), or a location of the occlusion within the vessel (e.g. in a proximal position, in a distal position, within a specified distance of a bifurcation, and so forth). In general, the measurement of a shape of the occlusion may represent an amount of bending or "tortuosity" of the occlusion, or a "entry shape" of the occlusion, i.e. the shape of its cap. The amount of bending may be defined in terms of the bending at the entry of the occlusion, as illustrated in Fig. 1C, or the bending along the path of the occlusion, as illustrated in Fig. 1D. Various examples of the "entry shape" of the occlusion are illustrated in Fig. IE, which illustrates a tapered cap to the occlusion, and Fig. IF, which illustrates a blunt cap to the occlusion. In general, the measurement of the composition of the occlusion may indicate the presence or distribution of various constituents in the composition. For example, the measurement of the composition of the occlusion may indicate the presence of different constituents (e.g. calcium) that are present in the occlusion, or the presence of type of plaque (e.g. calcified, soft plaque, hard plaque) in the occlusion, or the distribution (e.g. spotty, elongated) of a type of plaque in the occlusion, as illustrated in Fig. 1G.

In general, the operation of analyzing S120 the CT data to determine the one or more properties of an occlusion 120 in the vessel is performed using known image processing techniques, and based on differences in X-ray attenuation in the CT data. As mentioned above, the CT data that is received in the operation S110, and which is subsequently analyzed in the operation S120, may be generated by various types of X-ray imaging systems, including conventional CT imaging systems, and spectral CT imaging systems. Thus, the CT data may represent X-ray attenuation in a single energy interval, or it may be spectral CT data that represents X-ray attenuation in multiple different energy intervals.

The X-ray attenuation data from conventional CT imaging systems is typically expressed in Hounsfield Units. CT data representing X-ray attenuation in a single energy interval can be used to distinguish between the occlusion and surrounding media by virtue of differences in their attenuation. For instance, blood in the vessel has a lower X-ray attenuation coefficient than the occlusion, and consequently the lumen of a non-occluded portion of the vessel that contains blood may be distinguished from the occlusion. This facilitates a measurement of properties of the occlusion such as its size, location, and shape. A distinction may also be made between hard plaque and soft plaque in the occlusion based on differences in their X-ray attenuation coefficients, thereby facilitating a determination of the composition of the occlusion.

In one example, the CT data that is received in the operation S 110 is spectral CT data defining X-ray attenuation of the occluded vessel in a plurality of different energy intervals DE_{1..m}. In this example, the operation of analyzing S120 the CT comprises analyzing the spectral CT data to determine the one or more properties of the occlusion 120 in the vessel. As mentioned above, the use of spectral CT data facilitates a distinction to be made between media that have similar X-ray attenuation values when measured within a single energy interval, and which would be indistinguishable in X-ray attenuation data generated by a conventional X-ray imaging system. Thus, the use of spectral CT data in this example facilitates an improved distinction to be made between the X-ray attenuation arising from the occlusion, and the X-ray attenuation arising from other media such as contrast agent, dense tissue, bone, and so forth, that may also be represented in the spectral CT data.

In a related example, the one or more properties of the occlusion 120 that are determined in the operation S120 include a composition of the occlusion. In this example, the operation of analyzing S 120 the spectral CT comprises applying a material decomposition algorithm to the spectral CT data to determine the composition of the occlusion. In this example, the composition of the occlusion may indicate the presence of various materials, or various types of materials, in the occlusion. By way of some examples, the composition of the occlusion may indicate a type of plaque in the occlusion, such as calcified plaque, soft plaque, or hard plaque. Various material decomposition algorithms may be applied to the spectral CT data in this example. One example of a material decomposition algorithm that may be used is disclosed in a document by Brendel, B. et al., "Empirical, projection-based basis-component decomposition method", Medical Imaging 2009, Physics of Medical Imaging, edited by Ehsan Samei and Jiang Hsieh, Proc. of SPIE Vol. 7258, 72583Y. Another example of a material decomposition algorithm that may be used is disclosed in a document by Roessl, E. and Proksa, R., "K-edge imaging in X-ray computed tomography using multi-bin photon counting detectors", Phys Med Biol. 2007 Aug 7, 52(15):4679-96. Another example of a material decomposition algorithm that may be used is disclosed in the published PCT patent application WO/2007/034359 A2.

In some examples, the operation of analyzing S120 the CT data comprises reconstructing an image representing the occluded vessel 110. In these examples, the operation of analyzing S120 the CT data, is performed using the reconstructed image.

Various known image reconstruction techniques may be used to reconstruct the image in accordance with this example. The image may also be segmented in order to identify the occlusion. Various known segmentation algorithms may be used for this purpose, including model-based segmentation, watershed-based segmentation, region growing, level sets, graphcuts, and so forth. A neural network may also be trained to segment the reconstructed image in order to identify the occlusion.

In one example, the occlusion is automatically identified in the reconstructed image. In this example, the method described with reference to Fig. 3 includes:
segmenting the reconstructed image to identify the occlusion 120; and
automatically identifying the occlusion in the reconstructed image; and
wherein the analyzing S 120 the CT data to determine one or more properties of an occlusion 120 in the vessel, is performed on the identified occlusion.

By automatically identifying the occlusion, this example facilitates a faster determination of the recommended intravascular treatment device in the later operation S130. The segmentation operation in this example may be performed using the segmentation techniques described above. The operation of automatically identifying the occlusion in the reconstructed image may be performed using various known image processing techniques. For example, a feature detector, or a neural network, may be trained to identify the occlusion. A feature detector may for instance identify the occlusion based on an anomalous change of intensity in the vessel in the reconstructed image. Such changes may be attributed to changes in attenuation due to the transition between vessel that contains blood, and a vessel that contains occlusion material. If the CT data is acquired subsequent to the injection of a contrast agent, the magnitude of the transition is enhanced in the CT data, which facilitates an improved identification of the occlusion. Alternatively, a neural network such as a convolutional neural network, CNN, may be trained to identify the occlusion in the reconstructed image using training data that includes multiple reconstructed images representing a variety of occlusions and in which the corresponding ground truth location of the occlusion is annotated.

In another example, rather than being identified automatically in the reconstructed image, the occlusion is identified in the reconstructed image based on user input. In this example, the method described with reference to Fig. 3 includes:
outputting the reconstructed image to a display device 230, 330; and
receiving user input identifying the occlusion 120 in the reconstructed image; and
wherein the analyzing S 120 the CT data to determine one or more properties of an occlusion 120 in the vessel, is performed on the identified occlusion.

In this example, the reconstructed image may be outputted to a display device. The reconstructed image may be outputted to the monitor 230 illustrated in Fig. 4, for example. The user may identify the occlusion 120 in the reconstructed image using various user input devices such as a touchscreen, a pointing device such as a mouse, a joystick, a keyboard, and so forth. The user may identify the occlusion by delineating the occlusion using the user input device, or by placing a bounding box around the occlusion, for example.

Returning to the method illustrated in Fig. 3, in the operation S130, a recommended intravascular treatment device 130_{1..6} for treating the occluded vessel 110, is determined based on the one or more properties of the occlusion in the vessel. The recommended intravascular treatment device that is determined in the operation S130 may be specified at various levels, including for example at the level of a type of device, or at a lower level, such as a model of a type of device. Examples of different types of devices that may be determined in accordance with this example include the devices illustrated in Fig. 2, i.e.: a laser atherectomy device, a rotational atherectomy device, an orbital atherectomy device, a transluminal atherectomy device, and an IVL balloon. Since the recommended intravascular treatment device is determined based on the one or more properties of the occlusion, the treatment device is suited to the occlusion that is to be treated. Thus, a more optimal choice of treatment device can be made.

The operation S130 may be performed in various ways. These include: using a deterministic set of rules, using a lookup table, and using a neural network. In the first two of these approaches, the one or more properties of the occlusion are matched to an intravascular treatment device using rules that are determined by experts. Examples of such rules are described in the document by Dash, D., et al., cited above. In the third approach, such rules may be used to train a neural network to predict a recommended intravascular treatment device for a given set of one or more properties of the occlusion. By way of an example, the property/ properties of the occlusion may categorize the occlusion as including superficial calcium, deep calcium, or nodular calcium. Superficial calcium is typically defined as a calcified nodule that is located at the intimal-lumen interface or close to the lumen of the vessel. Deep calcium is typically defined as a calcified nodule that is located at the media/adventitia border or close to the adventitia. Nodular calcium is typically defined as the presence of multiple calcified nodules within the occlusion. If deep calcium is present, the recommended type of intravascular treatment device may be a laser atherectomy device. By contrast, if superficial calcium is present, and the calcium thickness exceeds 0.5 millimeters, and the length exceeds 5 millimeters, and its rotational angle exceeds 180 degrees around the centerline of the vessel, the recommended type of intravascular treatment device may be an IVL balloon. If nodular calcium is present, the recommended type of intravascular treatment device may be an orbital atherectomy device, or a rotational atherectomy device. Similar rules that depend on these and other properties of the occlusion may also be defined for other types of intravascular treatment device, or for specific models of such devices.

In another example, the operations of analyzing S 120 the CT data to determine one or more properties of an occlusion 120 in the vessel, and determining S130, based on the one or more properties, a recommended intravascular treatment device 130_{1..6} for treating the occluded vessel 110, are performed by a neural network. In this example, the CT data that is received in the operation S 110 is inputted into the neural network, and the neural network is trained to predict the recommended intravascular treatment device 130_{1..6} for treating the occluded vessel from the inputted CT data. The neural network is trained to predict the recommended intravascular treatment device 130_{1..6} using training data that includes a plurality of CT training images representing occluded vessels. Each CT training image includes a corresponding ground truth recommended intravascular treatment device for the vessel represented in the image. The ground truth recommended intravascular treatment device may be determined by an expert. The neural network may be provided by various types of architectures, including a convolutional neural network, CNN, architecture, for example. In this example, properties of the occlusion such as its size and shape are inherently determined by the neural network during its evaluation of the inputted CT data, although these features are not necessarily outputted by the neural network.

Returning to the method illustrated in Fig. 3, in the operation S140, an indication of the recommended intravascular treatment device 130_{1..6} is outputted. The outputting of the recommended intravascular treatment device provides guidance for the treatment procedure. The indication of the recommended intravascular treatment device may be outputted in any human-intelligible format. In one example, the indication of the recommended intravascular treatment device is outputted in a visual format. For instance, the indication of the recommended intravascular treatment device may be outputted to a display. The indication of the recommended intravascular treatment device may be outputted to the display 230 illustrated in Fig. 4, for example. The indication may be provided as a symbol or text that corresponds to the recommended intravascular treatment device, for example. The guidance information may alternatively be outputted in other ways, including audially, or to a printer, for example.

The properties of the vessel can also impact the choice of intravascular treatment device for treating an occlusion. For instance, the vessel diameter, the vessel tortuosity, the amount of contrast agent in the vessel, the amount of calcium in the vessel, the type and shape of plaque in the vessel, and the type and shape of plaque in the vessel along an access route to the occlusion, can also impact the choice of intravascular treatment device. In one example, the method described with reference to Fig. 3 includes:
analyzing the CT data to determine one or more properties of the vessel; and
the determining S130 a recommended intravascular treatment device 130_{1..6} for treating the occluded vessel 110, is based further on the one or more properties of the vessel.

Thus, in this example the recommended intravascular treatment device is determined based on the one or more properties of the vessel as well as the one or more properties of the occlusion. Thus improves the suitability of the recommended treatment device. In this example, the one or more properties of the vessel that are determined may include one or more of: a measurement of a size of the vessel (e.g. a measurement of a length of the vessel, or a diameter of a lumen of the vessel, or an area of a lumen of the vessel), a measurement of a shape of the vessel (e.g. a measure of tortuosity of the vessel path, or a measure of the shape of the vessel lumen), a measurement of an amount of plaque in the vessel, an identification of a type of plaque in the vessel, an identification of a shape of plaque in the vessel, and an identification of a composition of a plaque in the vessel. For instance, the size of the vessel lumen on proximal and/or distal sides of the occlusion affects its navigation in the vessel, and may be used to determine the recommended intravascular treatment device.

The properties of the vessel may be determined from the CT data in a similar manner to that described above for the occlusion. Thus, in general, the operation of analyzing the CT data to determine the one or more properties of the vessel is performed using known image processing techniques, and based on differences in X-ray attenuation in the CT data. The CT data may represent X-ray attenuation data in a single energy interval, or alternatively the CT data may be spectral CT data that defines X-ray attenuation in a plurality of different energy intervals. The use of spectral CT data facilitates an improved distinction to be made between the vessel, and other media such as dense tissue, and bone that may also be represented in the spectral CT data. The spectral CT data may be analyzed using a material decomposition algorithm, as mentioned above.

In this example, the operation of determining S130 a recommended intravascular treatment device 130_{1..6} for treating the occluded vessel 110 based further on the one or more properties of the vessel, may be performed in a similar manner to that described above for the property(ies) of the occlusion. Thus, a deterministic set of rules, or a lookup table, or a neural network, may be used. In the first two of these approaches, the one or more properties of the vessel are used in addition to the one or more properties of the occlusion, in order to find a matching intravascular treatment device, using the deterministic set of rules, or the lookup table. In the third of these approaches, the rules may be used to train a neural network to predict a recommended intravascular treatment device from an inputted set of one or more properties of the vessel as well as an inputted set of one or more properties of the occlusion.

In a related example, the operation of analyzing S120 the CT data comprises reconstructing an image representing the occluded vessel 110, and the operation of analyzing S120 the CT data, is performed using the reconstructed image. The centerline of the vessel may also be identified in the reconstructed image. The centerline may be used to determine the properties of the vessel, such as its size, shape, for example. The centerline may be determined by segmenting the lumen of the vessel in the reconstructed image, and defining the centerline of the vessel as the centerline of the lumen. The centerline of the lumen may be considered to provide an accurate position of the centerline of the vessel, and thus facilitates an accurate measurement of the properties of the vessel, such as its length, its amount of bending, and so forth. The centerline of the vessel may also be used to provide a more accurate measurement of the properties of the occlusion, such as its length, its amount of bending, and so forth.

In one example, the recommended intravascular treatment device 130_{1..6} is a recommended type of atherectomy device. With reference to Fig. 3, in this example, the operation of determining S130 a recommended intravascular treatment device 130_{1..6} for treating the occluded vessel 110, comprises determining a type of atherectomy device for treating the occluded vessel; and the operation of outputting S140 an indication of the recommended intravascular treatment device 130_{1..6}, comprises outputting an indication of the determined type of atherectomy device.

As mentioned above, after having selected a treatment device to treat an occlusion, it can be challenging to determine the properties of the device. Properties of the device such as its stiffness, and its tip shape, can also influence the effectiveness of the treatment, and consequently its outcome.

In one example, the method described with reference to Fig. 3 includes:
determining a recommended value of one or more properties of the recommended intravascular treatment device 130_{1..6}; and
outputting an indication of the recommended value of the one or more properties.

The values of various properties of the device may be recommended in accordance with this example, including for example a size of the device (e.g. a diameter, or a length of a plaque removal element of the device), a size of a guide catheter to use with the device, a stiffness of the device, a tip shape of the device, a tip load of the device, a coating of the device, and so forth. The recommended values of the properties may be determined using a deterministic set of rules, or by using a lookup table, or by using a neural network, in a similar manner to that described above in relation to the operation S130. The recommended values of the properties may be outputted in a similar manner to the indication of the recommended intravascular treatment device 130_{1..6}. Since in this example the recommended value of one or more properties is determined for the treatment device, the treatment device is more suited to the occlusion that is to be treated. Thus, a more effective treatment can be delivered.

As mentioned above, it can also be challenging to determine the values of treatment parameters to use with the recommended device. For instance, if the recommended device is a laser atherectomy catheter as illustrated in Fig. 2A, the values of treatment parameters such as a fluence of optical irradiation emitted by the device, and the rate of its advancement in the artery, may need to be adjusted in order to optimize the desired outcome. In one example, the method described with reference to Fig. 3 includes:
determining a recommended value of one or more treatment parameters of the recommended intravascular treatment device 130_{1..6}; and
outputting an indication of the recommended value of the one or more treatment parameters.

The values of various treatment parameters may be determined in this example, depending on the type of intravascular treatment device that is recommended. By way of an example, if the intravascular treatment device is a laser atherectomy catheter, the values of the treatment parameters that are recommended may include values for one or more of: a location of the laser atherectomy catheter with respect to the occlusion, an advancement speed of the laser atherectomy catheter, a fluence of optical irradiation emitted by the laser atherectomy catheter, a repetition rate of optical pulses emitted by the laser atherectomy catheter, and a duty cycle of optical irradiation emitted by the laser atherectomy catheter.

By way of another example, if the intravascular treatment device is a directional atherectomy device, the values of the treatment parameters that are recommended may include values for one or more of: a location of the directional atherectomy device with respect to the occlusion, an advancement speed of the directional atherectomy device, and a rotation speed of the cutting window of the directional atherectomy device.

By way of another example, if the intravascular treatment device is a rotational atherectomy device, the values of the treatment parameters that are recommended may include values for one or more of: a location of the rotational atherectomy device with respect to the occlusion, an advancement speed of the rotational atherectomy device, and a rotation speed of the burr of the rotational atherectomy device.

By way of another example, if the intravascular treatment device is an orbital atherectomy device, the values of the treatment parameters that are recommended may include values for one or more of: a location of the orbital atherectomy device with respect to the occlusion, an advancement speed of the orbital atherectomy device, and a rotation speed of the orbital atherectomy device.

By way of another example, if the intravascular treatment device is a transluminal atherectomy device, the values of the treatment parameters that are recommended may include values for one or more of: a location of the transluminal atherectomy device with respect to the occlusion, an advancement speed of the transluminal atherectomy device, and an aspiration flow rate of the transluminal atherectomy device.

By way of another example, if the intravascular treatment device is an intravascular lithotripsy, IVL, balloon, the at least one parameter may include one or more of: a location of the IVL balloon with respect to the occlusion, a number of shock wave pulses to deliver to the occlusion from one or more shock wave emitters of the IVL balloon, and an IVL balloon pressure to use during a delivery of shock waves to the occlusion from the IVL balloon.

The recommended values of the treatment parameters may be determined using a deterministic set of rules, or by using a lookup table, or by using a neural network, i.e. in a similar manner to that described above in relation to the operation S130. The recommended values of the treatment parameters may be outputted in a similar manner to the indication of the recommended intravascular treatment device 130_{1..6}. By providing the recommended value of one or more treatment parameters of the intravascular treatment device, this example facilitates the delivery of a more effective treatment to the occlusion.

In one example, the recommended values of the treatment parameters are used to automatically adjust settings of an intravascular treatment device 130_{1..6}. This improves workflow by avoiding the need for time-consuming user adjustments of the treatment parameters.

In one example, the values of the treatment parameters are synchronized with a cardiac phase of a subject. For example, treatment parameters such as the fluence of optical irradiation emitted by a laser atherectomy catheter, may be synchronized with the cardiac phase such that optical irradiation is only emitted during a diastole phase. This improves the effectiveness of delivering treatment to the occlusion because the curvature of the vessel in which the occlusion is disposed is typically more variable in the systole phase than in the diastole phase. The cardiac phase of the subject may be determined using a sensor. For example, an electrocardiogram "EKG" sensor may be used.

In another example, an indication of one or more recommended procedural steps to be performed with the recommended intravascular treatment device, is outputted. In this example, the method described with reference to Fig. 3 includes:
determining one or more recommended procedural steps to be performed with the recommended intravascular treatment device 130_{1..6}; and
outputting an indication of the one or more recommended procedural steps.

Examples of recommended procedural steps that may be outputted in this example include "advance treatment device at (a specified) rate", "withdraw treatment device (a specified distance), rotate treatment device through (a specified) angle, then re-advance tool", "obtain intravascular ultrasound "IVUS" imaging data for the occluded vessel", and so forth. The recommended procedural step(s) may be determined in accordance with the rules disclosed in a document by Brilakis, E., et al., "Guiding Principles for Chronic Total Occlusion Percutaneous Coronary Intervention: A Global Expert Consensus Document", Circulation, 2019; 140:420-433.

The recommended procedural steps may include a recommended CTO crossing strategy such as "Antegrade Wiring", "Antegrade Dissection and Re-entry", "Retrograde Wiring", or "Retrograde Dissection and Re-entry". The crossing strategy may be determined based on the occlusion properties and/or the vessel properties. The crossing strategy may be determined in accordance with the so-called Hybrid algorithm disclosed in the document by Brilakis, E., et al., "A percutaneous treatment algorithm for crossing coronary chronic total occlusions", JACC Cardiovasc Interv. 2012;5:367-379.

The recommended procedural steps may be determined in a similar manner to that described above for determining the type of atherectomy device to use, i.e. using a deterministic set of rules, or a lookup table, or a neural network.

In another example, the method described with reference to Fig. 3 includes:
determining a value of an outcome metric for the treatment procedure; and
outputting an indication of the value of the outcome metric.

The value of the outcome metric may represent various factors. For instance, the value of the outcome metric may represent a probability of success or failure of the procedure, or a duration of the procedure, or the probability of a medical complication arising from the procedure.

The outcome metric may be evaluated in various ways. In one example, the so-called J-CTO score is used. The J-CTO score estimates the likelihood of successful antegrade guidewire crossing of a CTO within 30 minutes. The evaluation of the J-CTO score is described in a document by Morino, Y., et al., "Predicting Successful Guidewire Crossing Through Chronic Total Occlusion of Native Coronary Lesions Within 30 Minutes: The J-CTO (Multicenter CTO Registry in Japan) Score as a Difficulty Grading and Time Assessment Tool", JACC: Cardiovascular Interventions, Volume 4, Issue 2, February 2011, Pages 213-221. The J-CTO score is evaluated based on five criteria: the entry shape (tapered vs blunt) of the occlusion, the presence of calcification within the CTO, the presence of at least one bend exceeding 45 degrees at the CTO entry, or along the CTO body, the length of the occlusion, and whether the current attempt is a repeated attempt. The J-CTO score may be evaluated from the properties of the occlusion described above with reference to Fig. 1.

In another example, the value of the outcome metric is determined from a database of historic procedures that have been performed on occlusions using intravascular treatment devices, and for which the values of corresponding outcome metrics have been recorded. The value of the outcome metric may be determined from the database based on the value of the outcome metric for procedures that have been performed using similar intravascular treatment devices, and on similar occlusions. One or more additional factors may also be used to determine the value of the outcome metric, such as for example a similarity in the properties of the intravascular treatment device used in the procedure, or a similarity in the treatment parameters of the intravascular treatment device used in the procedure. In his example, the similarity between the procedures, devices, and so forth may be calculated using a similarity metric such as the Mahalanobis distance, for example. In this example, the value of the outcome metric for the most similar historic procedure may be used as the value of the outcome metric for the current procedure.

In another example, the value of the outcome metric is determined using a geometric model that is generated from the CT data that is received in the operation S110. In this example, the operation of determining a value of an outcome metric for the treatment procedure comprises:
extracting from the CT data, geometric data representing the vessel and the occlusion 120;
generating, from the geometric data, a geometric model representing the vessel and the occlusion 120;
   determining an expected effect of the recommended intravascular treatment device on the occluded vessel 110 using the geometric model; and
determining the value of the outcome metric for the treatment procedure based on the expected effect of the recommended intravascular treatment device 130_{1..6} on the occluded vessel.

In this example, the extraction of geometric data representing the vessel and the occlusion 120 from the CT data may be performed in a similar manner to that described above for the analyzing the CT data in the operation S120 to determine the one or more properties of an occlusion 120 in the vessel. Thus, if CT data representing X-ray attenuation in a single energy interval is used, the CT data may be reconstructed into an image, and the image may be segmented in order to identify the vessel and the occlusion. The geometric data may then be extracted from the reconstructed image using image processing techniques. If spectral CT data is used a material decomposition algorithm may be applied to the spectral CT data in order to identify the vessel and the occlusion, during the reconstruction of an image representing the vessel and the occlusion. The geometric data may then be extracted from the reconstructed image using known image processing techniques.

After having extracted the geometric data, the geometric model representing the vessel and the occlusion 120 is then generated from the geometric data. The geometric model may be provided in the form of a finite element model. Examples of such a finite element model is disclosed in a document by Holzapfel, G., et al., "Computational approaches for analyzing the mechanics of atherosclerotic plaques: A review", Journal of Biomechanics, Volume 47, Issue 4, 3 March 2014, Pages 859-869. The expected effect of the recommended intravascular treatment device on the occluded vessel 110 may then be determined using the geometric model by adapting the model by removing material by the treatment device in accordance with the delivery of treatment. The value of the outcome metric for the treatment procedure is then determined based on the expected effect of the recommended intravascular treatment device 130_{1..6} on the occluded vessel. By way of an example, the outcome metric may be evaluated based on amount of increase in the lumen diameter as a result of the treatment, i.e. the so-called "lumen gain".

In a related example, the geometric model is a biomechanical model, and the CT data represents a temporal sequence of images representing the occluded vessel 110. In this example, the method described with reference to Fig. 3 includes determining one or more biomechanical parameters of the biomechanical model based on temporal changes to a shape of the vessel in the temporal sequence of images representing the occluded vessel.

In this example, the biomechanical parameters of the biomechanical model may represent mechanical parameters such as the stiffness of the vessel and the occlusion. The biomechanical model provides a more accurate characterization of the deformation of the vessel and the occlusion in response to the delivery of the treatment. Consequently, the biomechanical model provides a more accurate prediction of the effect the intravascular treatment device. This facilitates the determination of a more accurate value of the outcome metric.

In another example, the method described with reference to Fig. 3 is used contemporaneously with an occlusion treatment procedure. In this example, the method described with reference to Fig. 3 includes:
receiving X-ray projection data representing the occluded vessel, the X-ray projection data being generated during the treatment procedure;
registering the CT data to the X-ray projection data; and
outputting a graphical representation of the X-ray projection data and the CT data as an overlay image.

This example may be performed by the system 300 illustrated in Fig. 5, and which includes an X-ray projection imaging system 320 for generating the X-ray projection data. The X-ray projection data may represent a static image, of the occluded vessel or a temporal sequence of images of the occluded vessel. In the latter case, the temporal sequence of images may represent the occluded vessel substantially in real-time. The operations of receiving X-ray projection data, registering the CT data to the X-ray projection data, and outputting the graphical representation of the X-ray projection data and the CT data, may be performed substantially in real-time in order to provide live guidance during the treatment procedure. In this example, the X-ray projection data may be received by the one or more processors 210 illustrated in Fig. 5.

In this example, the CT data is registered to the X-ray projection data using known image registration techniques. The registration may be performed using the known geometry of the X-ray projection imaging system and its orientation with respect to the CT data. Examples of suitable image registration techniques include intensity-based registration techniques, feature-based registration techniques, rigid and non-rigid registration techniques, and so forth. A graphical representation of the X-ray projection data and the CT data is then outputted as an overlay image. The registration is used to generate the overlay image. This operation may be performed using known image overlay techniques. For instance, one image from the X-ray projection data and the CT data may be overlaid on the other image, wherein the overlaid image has a predetermined level of transparency. The graphical representation may be outputted in various ways, such as to display, for example. The graphical representation may be outputted to the display 330 illustrated in Fig. 5, for example. The outputted overlay image provides guidance to a physician during the treatment procedure by providing three-dimensional context from the CT data, to the planar representation of the occluded vessel from the X-ray projection data

In a related example, the method described with reference to Fig. 3 includes:
receiving input data representing a delivery of a treatment to the occlusion by an intravascular treatment device 130_{1..6} during the treatment procedure; and
updating the overlay image based on the received input data; and
wherein the updating comprises including in the overlay image an indication of the delivery of the treatment to the occlusion.

This example may be performed by tracking the position of the intravascular treatment device with respect to the X-ray projection data that is generated during the treatment procedure, and indicating the tracked position of the intravascular treatment device in the overlay image for the times at which the treatment is delivered to the vessel. By way of an example, the indication of the delivery of the treatment to the occlusion may indicate that a specified number of optical pulses have been delivered to the occlusion by the laser atherectomy device illustrated in Fig. 2A. The indication of the delivery of the treatment to the occlusion may be provided in the overlay image as a symbol at the relevant position, for example. The tracked position of the treatment device may be determined in the X-ray projection data, or using a separate tracking system. In the former case, the tracked position of the intravascular treatment device may be determined in the X-ray projection image using a feature detector that is trained to detect the shape of the intravascular treatment device. Alternatively, a feature detector may be trained to detect the shape of a fiducial marker that is attached to the treatment device. In the latter case, various interventional device tracking techniques may be used to track the position of the intravascular treatment device. The position may be determined in the X-ray projection image by registering the coordinate system of the tracking system to the coordinate system of the X-ray projection imaging system 320 that generates the X-ray projection data. Various tracking systems may be used to track the position of the intravascular treatment device, including electromagnetic tracking systems and optical fiber-based tracking systems. An example of an electromagnetic tracking system is disclosed in the document US 2020/397510 A1. An example of an optical fiber-based tracking technique that uses a strain sensor to determine the position of an interventional device is disclosed in the document US 2012/323115 A1.

In this example, the received input data may represent a position of the delivery of the treatment to the occlusion. The operation of updating the overlay image may include providing in the overlay image an indication of the position of the delivery of the treatment to the occlusion. Alternatively, or additionally, the operation of updating the overlay image may include providing in the overlay image an indication of a total number of treatments delivered to the occlusion. For example, the total number of optical pulses have been delivered to the occlusion by the laser atherectomy device illustrated in Fig. 2A, may be indicated in the overlay image. Thus, the overlay image provides a record of the treatment that has been delivered to the occlusion.

In another example, a computer program product is provided. The computer program product comprises instructions which when executed by one or more processors, cause the one or more processors to carry out a method of providing guidance for a treatment procedure on an occluded vessel 110. The method includes:
receiving S110 computed tomography, CT, data representing the occluded vessel 110;
analyzing S120 the CT data to determine one or more properties of an occlusion 120 in the vessel;
determining S130, based on the one or more properties, a recommended intravascular treatment device 130_{1..6} for treating the occluded vessel 110; and
outputting S140 an indication of the recommended intravascular treatment device 130_{1..6}.

In another example, a system 200, 300 for providing guidance for a treatment procedure on an occluded vessel 110, is provided. The system comprises one or more processors 210 configured to:
receive S110 computed tomography, CT, data representing the occluded vessel 110;
analyze S120 the CT data to determine one or more properties of an occlusion 120 in the vessel;
determine S130, based on the one or more properties, a recommended intravascular treatment device 130_{1..6} for treating the occluded vessel 110; and
output S140 an indication of the recommended intravascular treatment device 130_{1..6}.

One example of the system 200 is illustrated in Fig. 4. This example may be used to provide guidance for the planning phase of an occlusion treatment procedure. It is noted that the system 200 illustrated in Fig. 4 may also include one or more of: a CT imaging system 220 for providing the CT data 140, a display 230 for displaying a graphical representation of the CT data, the outputted results of the method such as the property(ies) of the occlusion 120, the property(ies) of the vessel, and so forth; a patient bed 240; and a user input device configured to receive user input (not illustrated in Fig. 4) in relation to the method performed by the one or more processors 210, such as a keyboard, a mouse, a touchscreen, and so forth.

Another example of the system 300, is illustrated in Fig. 5. This example may be used to provide guidance for the treatment phase of an occlusion treatment procedure. It is noted that the system 300 illustrated in Fig. 5 may also include one or more of: an X-ray projection imaging system 320 for providing X-ray projection data, a display 330 for displaying a graphical representation of the X-ray projection data and the CT data as an overlay image, and further outputted results of the method such as the property(ies) of the occlusion 120, the property(ies) of the vessel, and so forth; a patient bed 340; and a user input device configured to receive user input (not illustrated in Fig. 5) in relation to the method performed by the one or more processors 210, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the corresponding computer program product, or by the corresponding computer-readable storage medium, or by the corresponding system. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of providing guidance for a treatment procedure on an occluded vessel (110), the method comprising:
receiving (S110) computed tomography, CT, data representing the occluded vessel (110);
analyzing (S120) the CT data to determine one or more properties of an occlusion (120) in the vessel;
determining (S130), based on the one or more properties, a recommended intravascular treatment device (130_{1..6}) for treating the occluded vessel (110); and
outputting (S140) an indication of the recommended intravascular treatment device (130_{1..6}).

2. The computer-implemented method according to claim 1, wherein the one or more properties of the occlusion (120) include one or more of: a measurement of a size of the occlusion, a location of the occlusion, a measurement of a shape of the occlusion, a measurement of an entry shape of the occlusion, and a composition of the occlusion.

3. The computer-implemented method according to claim 1 or claim 2, wherein the CT data comprises spectral CT data defining X-ray attenuation of the occluded vessel in a plurality of different energy intervals (DE_{1..m}); and
wherein the analyzing (S120) the CT comprises analyzing the spectral CT data to determine the one or more properties of the occlusion (120) in the vessel.

4. The computer-implemented method according to claim 3, wherein the one or more properties of the occlusion (120) include a composition of the occlusion; and
wherein the analyzing (S120) the spectral CT comprises applying a material decomposition algorithm to the spectral CT data to determine the composition of the occlusion.

5. The computer-implemented method according to any previous claim, wherein the analyzing (S120) the CT data comprises:
reconstructing an image representing the occluded vessel (110); and
wherein the analyzing (S120) the CT data, is performed using the reconstructed image.

6. The computer-implemented method according to claim 5, wherein the method further comprises:
segmenting the reconstructed image to identify the occlusion (120); and
automatically identifying the occlusion in the reconstructed image; and
wherein the analyzing (S120) the CT data to determine one or more properties of an occlusion (120) in the vessel, is performed on the identified occlusion.

7. The computer-implemented method according to claim 5, wherein the method further comprises:
outputting the reconstructed image to a display device (230, 330); and
receiving user input identifying the occlusion (120) in the reconstructed image; and
wherein the analyzing (S120) the CT data to determine one or more properties of an occlusion (120) in the vessel, is performed on the identified occlusion.

8. The computer-implemented method according to any previous claim, wherein the method further comprises:
analyzing the CT data to determine one or more properties of the vessel; and
wherein the determining (S130) a recommended intravascular treatment device (130_{1..6}) for treating the occluded vessel (110), is based further on the one or more properties of the vessel.

9. The computer-implemented method according to claim 8, wherein the one or more properties of the vessel include one or more of: a measurement of a size of the vessel, a measurement of a shape of the vessel, a measurement of an amount of plaque in the vessel, a type of plaque in the vessel, a shape of plaque in the vessel, and a composition of a plaque in the vessel.

10. The computer-implemented method according to any previous claim, wherein the determining (S130) a recommended intravascular treatment device (130_{1..6}) for treating the occluded vessel (110), comprises determining a type of atherectomy device for treating the occluded vessel; and
wherein the outputting (S140) an indication of the recommended intravascular treatment device (130_{1..6}), comprises outputting an indication of the determined type of atherectomy device.

11. The computer-implemented method according to claim 10, further comprising:
determining a recommended value of one or more properties of the recommended intravascular treatment device (130_{1..6}); and
outputting an indication of the recommended value of the one or more properties.

12. The computer-implemented method according to claim 10 or claim 11, further comprising:
determining a recommended value of one or more treatment parameters of the recommended intravascular treatment device (130_{1..6}); and
outputting an indication of the recommended value of the one or more treatment parameters.

13. The computer-implemented method according claim 1 wherein the method further comprises:
determining a value of an outcome metric for the treatment procedure; and
outputting an indication of the value of the outcome metric.

14. The computer-implemented method according to any previous claim, wherein the method further comprises:
receiving X-ray projection data representing the occluded vessel, the X-ray projection data being generated during the treatment procedure;
registering the CT data to the X-ray projection data; and
outputting a graphical representation of the X-ray projection data and the CT data as an overlay image.

15. The computer-implemented method according to claim 14, wherein the method further comprises:
receiving input data representing a delivery of a treatment to the occlusion by an intravascular treatment device (130_{1..6}) during the treatment procedure; and
updating the overlay image based on the received input data; and
wherein the updating comprises including in the overlay image an indication of the delivery of the treatment to the occlusion.
